# EUROPEAN PATENT SPECIFICATION

(11) **EP 1 148 874 B1**
(45) Date of publication and mention of the grant of the patent: **10.12.2003**
(21) Application number: 00905841.3
(22) Date of filing: 31.01.2000
(51) Int. Cl.: A61K 31/35, A61K 31/36, A61K 31/18, A61P 43/00

(54) **USE OF ANTICONVULSANT DERIVATIVES FOR TREATING BULIMIA NERVOSA**
VERWENDUNG VON ANTIKONVULSIVEN DERIVATEN ZUR BEHANDLUNG VON BULIMIA NERVOSA
UTILISATION DE DERIVES ANTI-CONVULSIFS POUR TRAITER LA BOULIMIE NERVEUSE

(30) Priority: 01.02.1999 US 118057 P
(43) Date of publication of application: 31.10.2001
(73) Proprietor: Ortho-McNeil Pharmaceutical, Inc., Raritan, NJ 08869-0602 (US)
(72) Inventor: HOOPES, Scott, P., Eagle, ID 83616 (US)
(74) Representative: Fisher, Adrian John
(86) International application number: US0002334
(87) International publication number: WO00044374

(56) References cited:
- EP-A- 0 138 441
- WO-A-93/00083
- MARYANOFF B E ET AL: "ANTICONVULSANT O-ALKYL SULFAMATES. 2,3:4,5-BIS-O- (1-METHYLETHYLIDENE)-BETA-D-FRUCTOPYRANOSE SULFAMATE AND RELATED COMPOUNDS" JOURNAL OF MEDICINAL CHEMISTRY,US,AMERICAN CHEMICAL SOCIETY. WASHINGTON, vol. 30, no. 5, 1 May 1987 (1987-05-01), pages 880-887, XP000647869 ISSN: 0022-2623 cited in the application
- WAUQUIER A ET AL: "TOPIRAMATE: A POTENT ANTICONVULSANT IN THE AMYGDALA-KINDLED RAT" EPILEPSY RESEARCH,NL,ELSEVIER SCIENCE PUBLISHERS, AMSTERDAM, vol. 24, no. 2, 1 June 1996 (1996-06-01), pages 73-77, XP002042953 ISSN: 0920-1211 cited in the application

## Description

### BACKGROUND OF THE INVENTION

The application relates to the use of a compound of formula I for the manufacture of a medicament for treating bulimia nervosa in a subject.

Compounds of Formula I: 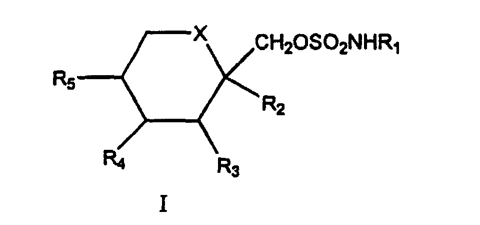 are structurally novel antiepileptic compounds that are highly effective anticonvulsants in animal tests (Maryanoff, B.E, Nortey, S.O., Gardocki, J.F., Shank, R.P. and Dodgson, S.P. *J. Med. Chem*. 30, 880-887, 1987; Maryanoff, B.E., Costanzo, M.J., Shank, R.P., Schupsky, J.J., Ortegon, M.E., and Vaught J.L. Bioorganic & Medicinal Chemistry Letters 3, 2653-2656, 1993). These compounds are covered by US Patent No.4,513,006. One of these compounds 2,3:4,5-bis-O-(1-methylethylidene)-β-D-fructopyranose sulfamate known as topiramate has been demonstrated in clinical trials of human epilepsy to be effective as adjunctive therapy or as monotherapy in treating simple and complex partial seizures and secondarily generalized seizures (E. FAUGHT, B.J. WILDER, R.E. RAMSEY, R.A. REIFE, L D. KRAMER, G.W. PLEDGER, R.M. KARIM et. al., Epilepsia 36 (S4) 33, 1995; S.K. SACHDEO, R.C. SACHDEO, R.A. REIFE, P. LIM and G. PLEDGER, Epilepsia 36 (S4) 33, 1995), and is currently marketed for the treatment of simple and complex partial seizure epilepsy with or without secondary generalized seizures in approximately twenty countries including the United States, and applications for regulatory approval are presently pending in several additional countries throughout the world.

Compounds of Formula I were initially found to possess anticonvulsant activity in the traditional maximal electroshock seizure (MES) test in mice (SHANK, R.P., GARDOCKI, J.F., VAUGHT, J.L., DAVIS, C.B., SCHUPSKY, J.J., RAFFA, R.B., DOIDGSON, S.J., NORTEY, S.O., and MARYANOFF, B.E., Epilepsia 35 450-460, 1994). Subsequent studies revealed that Compounds of Formula I were also highly effective in the MES test in rats. More recently topiramate was found to effectively block seizures in several rodent models of epilepsy (J. NAKAMURA, S. TAMURA, T. KANDA, A. ISHII, K. ISHIHARA, T. SERIKAWA, J. YAMADA, and M. SASA, Eur. J. Pharmacol. 254 83-89, 1994), and in an animal model of kindled epilepsy (A. WAUQUIER and S. ZHOU, Epilepsy Res. 24, 73-77, 1996).

Bulimia nervosa is characterized by recurrent episodes of binge eating associated with inappropriate compensatory behavior to prevent weight gain (Diagnostic Statistical Manual of the American Psychiatric Association IVth Edition). It is more common in females than males; self evaluation is unduly influenced by body shape and weight and occurs in persons with high rates of mood and impulse control disorders.

### DISCLOSURE OF THE INVENTION

Accordingly, it has been found that compounds of the following formula I: 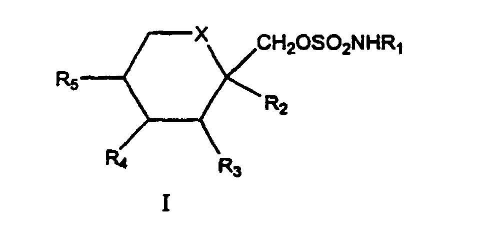 wherein X is O or CH₂, and R1, R2, R3, R4 and R5 are as defined hereinafter are useful in treating bulimia.

### DETAILED DESCRIPTION OF THE PREFERRED EMBODIEMENTS

The sulfamates of the invention are of the following formula (I): 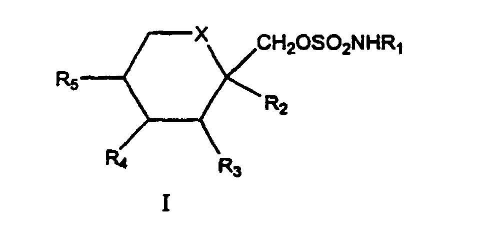 wherein
X is CH₂ or oxygen;
R₁ is hydrogen or C₁-C₄ alkyl; and
R₂, R₃, R₄ and R₅ are independently hydrogen or C₁-C₃ alkyl and, when X is CH₂, R₄ and R₅ may be alkene groups joined to form a benzene ring and, when X is oxygen, R₂ and R₃ and/or R₄ and R₅ together may be a methylenedioxy group of the following formula (II): 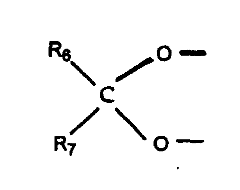 wherein
R₆ and R₇ are the same or different and are hydrogen, C₁-C₃ alkyl or are alkyl and are joined to form a cyclopentyl or cyclohexyl ring.

R₁ in particular is hydrogen or alkyl of about 1 to 4 carbons, such as methyl, ethyl, iso-propyl, *n*-propyl, *n*-butyl, isobutyl, *sec*-butyl and *t*-butyl. Alkyl throughout this specification includes straight and branched chain alkyl. Alkyl groups for R₂, R₃, R₄, R₅, R₆ and R₇ are of about 1 to 3 carbons and include methyl, ethyl, iso-propyl and n-propyl. When X is CH₂, R₄ and R₅ may combine to form a benzene ring fused to the 6-membered X-containing ring, i.e., R₄ and R₅ are defined by the alkatrienyl group =C-CH=CH-CH=.

A particular group of compounds of formula (I) is that wherein X is oxygen and both R₂ and R₃ and R₄ and R₅ together are methylenedioxy groups of the formula (II), wherein R₆ and R₇ are both hydrogen both alkyl or combine to form a spiro cyclopentyl or cyclohexyl ring, in particular where R₆ and R₇ are both alkyl such as methyl. A second group of compounds is that wherein X is CH₂ and R₄ and R₅ are joined to form a benzene ring. A third group of compounds of formula (I) is that wherein both R₂ and R₃ are hydrogen.

A particularly preferred compound for the use of the present invention is 2,3:4,5-bis-O-(1-methylethylidene)-β-D-fructopyranose sulfamate, known as topiramate. Topiramate has the following structural formula

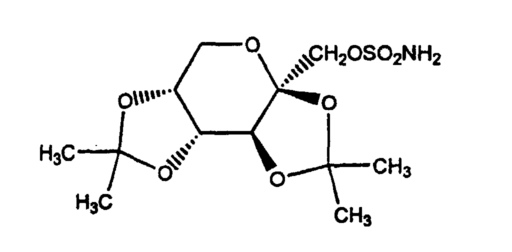

The compounds of formula (I) may be synthesized by the following methods:
(a) Reaction of an alcohol of the formula RCH₂OH with a chlorosulfamate of the formula CISO₂NH₂ or CISO₂NHR₁ in the presence of a base such as potassium a-butoxide or sodium hydride at a temperature of about -20° to 25° C and in a solvent such as toluene, THF or dimethylformamide wherein R is a moiety of the following formula (III): 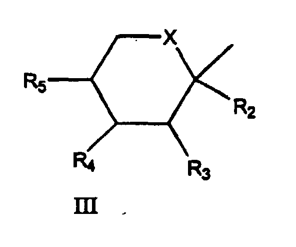
(b) Reaction of an alcohol of the formula RCH₂OH with sulfurylchloride of the formula SO₂Cl₂ in the presence of a base such as triethylamine or pyridine at a temperature of about -40° to 25° C in a solvent such as diethyl ether or methylene chloride to produce a chlorosulfate of the formula RCH₂OSO₂Cl.
   The chlorosulfate of the formula RCH₂OSO₂Cl may then be reacted with an amine of the formula R₁NH₂ at a temperature of abut 40° to 25° C in a solvent such as methylene chloride or acetonitrile to produce a compound of formula (I). The reaction conditions for (b) are also described by T. Tsuchiya et al. in Tet. Letters, No. 36, p. 3365 to 3368 (1978).
(c) Reaction of the chlorosulfate RCH₂OSO₂Cl with a metal azide such as sodium azide in a solvent such as methylene chloride or acetonitrile yields an azidosulfate of the formula RCH₂OSO2N₃ as described by M. Hedayatullah in Tet. Lett. p. 2455-2458 (1975). The azidosulfate is then reduced to a compound of formula (I) wherein R₁ is hydrogen by catalytic hydrogenation, e.g. with a noble metal and H₂ or by heating with copper metal in a solvent such as methanol.

The starting materials of the formula RCH₂OH may be obtained commercially or as known in the art. For example, starting materials of the formula RCH₂OH wherein both R₂ and R₃ and R₄ and R₅ are identical and are of the formula (II) may be obtained by the method of R. F. Brady in Carbohydrate Research, Vol. 14, p. 35 to 40 (1970) or by reaction of the trimethylsilyl enol ether of a R₆COR₇ ketone or aldehyde with fructose at a temperature of about 25° C, in a solvent such a halocarbon, e.g. methylene chloride in the presence of a protic acid such as hydrochloric acid or a Lewis Acid such as zinc chloride. The trimethylsilyl enol ether reaction is described by G. L. Larson et al in J. Org. Chem. Volaa 38, No. 22, p. 3935 (1973).

Further, carboxylic acids and aldehydes of the formulae RCOOH and RCHO may be reduced to compounds of the formula RCH2OH by standard reduction techniques, e.g. reaction with lithium aluminum hydride, sodium borohydride or boranc-THF complex in an inert solvent such a diglyme, THF or toluene at a temperature of about 0° to 100° C, e.g. as described by H.O. House in "Modern Synthetic Reactions", 2nd Ed., pages 45 to 144 (1972).

The compounds of formula I: may also be made by the processes disclosed in U.S. Patent Nos. 4,513,006, 5,387,700 and 5,387,700. More particularly, topiramate may be prepared following the process described in Examples 1 to 3 of U.S. 5,387,700.

The compounds of formula I include the various individual isomers as well as the racemates thereof, e.g., the various alpha and beta attachments, i.e., below and above the plane of the drawing, of R₂, R₃, R₄ and R₅ on the 6-membered ring. Preferably, the oxygen of the methylenedioxy group (II) are attached on the same side of the 6-membered ring.

The term "subject" as used herein, refers to an animal, preferably a mammal, most preferably a human, who has been the object of treatment, observation or experiment.

The term ''therapeutically effective amount" as used herein, means that amount of active compound or pharmaceutical agent that elicits the biological or medicinal response in a tissue system, animal or human that is being sought by a researcher, veterinarian, medical doctor or other clinician, which includes alleviation of the symptoms of the disease or disorder being treated.

It has been suggested that variations in brain GABA metabolism may be involved in the control of food intake in rats and that experimental brain GABA elevation produced anorexia in adult female rats (Coscina DV, *GABA and feeding: reversal of overeating by central GABA-transaminase inhibition*, Progress in Neuro-Psychopharmacology & Biological Psychiatry, 7(4-6):463-7,1983). It has also been shown that endogenous lateral hypothalamic glutamate acts to regulate natural eating and body weight in rats (Stanley BG, Willett VL 3^{rd}, Donias HW, Dee MG 2^{nd}, Duva MA, *Lateral hypothalamic NMDA receptors and glutamate as physiological mediators of eating and weight control*, American Journal of Physiology, 270(2Pt 2):R443-9, 1996 Feb.). Based on the known enhancement of brain GABA activity and reduced glutamate receptor activity of topiramate, we hypothesize that there is potential efficacy of topiramate in the treatment of bulimia.

For treating bulimia, a compound of formula (I) may be employed at a total daily dosage in the range of 15 mg to 500 mg, preferably, 16 mg to 200 mg, for an average adult human, administered one to four times per day, preferably, one to two times per day. A unit dose typically contains 16 to 300 mg, preferably, 16 to 200 mg, of the active ingredient.

Optimal dosages to be administered may be readily determined by those skilled in the art, and will vary with the particular compound used, the mode of administration, the strength of the preparation, the mode of administration, and the advancement of the disease condition. In addition, factors associated with the particular patient being treated, including patient age, weight, diet and time of administration, will result in the need to adjust dosages.

To prepare the pharmaceutical compositions of this invention, one or more sulfamate compounds of formula (I) are intimately admixed with a pharmaceutical carrier according to conventional pharmaceutical compounding techniques, which carrier may take a wide variety of forms depending on the form of preparation desired for administration, e.g., oral, by suppository, or parenteral. In preparing the compositions in oral dosage form, any of the usual pharmaceutical media may be employed. Thus, for liquid oral preparations, such as for example, suspensions, elixirs and solutions, suitable carriers and additives include water, glycols, oils, alcohols, flavoring agents, preservatives, coloring agents and the like; for solid oral preparations such as, for example, powders, capsules and tablets, suitable carriers and additives include starches, sugars, diluents, granulating agents, lubricants, binders, disintegrating agents and the like. Because of their ease in administration, tablets and capsules represent the most advantageous oral dosage unit form, in which case solid pharmaceutical carriers are obviously employed. If desired, tablets may be sugar coated or enteric coated by standard techniques. Suppositories may be prepared, in which case cocoa butter could be used as the carrier. For parenterals, the carrier will usually comprise sterile water, though other ingredients, for example, for purposes such as aiding solubility or for preservation, may be included. Injectable solutions may also be prepared in which case appropriate stabilizing agents may be employed. Topiramate is currently available for oral administration in round tablets containing 25 mg, 100 mg or 200 mg of active agent. The tablets contain the following inactive ingredients: lactose hydrous, pregelatinized starch, microcrystalline cellulose, sodium starch glycolate, magnesium stearate, purified water, carnauba wax, hydroxypropyl methylcellulose, titanium dioxide, polyethylene glycol, synthetic iron oxide, and polysorbate 80.

The pharmaceutical compositions herein will contain, per dosage unit, e.g., tablet, capsule, powder injection, teaspoonful, suppository and the like from 25 to 200 mg of the active ingredient.

The following Examples are set forth to aid in the understanding of the invention, and are not intended and should not be construed to limit in any way the invention set forth in the claims which follow thereafter.

### EXAMPLE 1

**Patient 01:** Patient started topiramate at a dose of 25 mg qhs and titrated by 25 mg/week to a dose of 100 mg/day. Within 1 month, patient had stopped bingeing for 2 weeks (had been bingeing daily) and felt control over her eating. After 5 months, patient was still bingeing and purging less, but has parasthesia so the timing of her topiramate was adjusted to try and relieve this. She has not lost weight although not gained either in spite of increased activity. She had run out of topiramate 1 week prior to this appointment and her mood had sharply deteriorated. Wellbutrin was added at this visit. Three weeks later, patient was feeling well, continued benefit to her bulimia, and parasthesias have resolved with topiramate 100 mg qd dosing.

### EXAMPLE 2

**Patient 02:** Patient started topiramate 25 mg qhs and titrated by 25 mg/week to dose of 100 mg/day. At first visit, she was purging 3 times/day to 3 times/week. After 3 weeks (topiramate 75 mg/hs), she is now satisfied when she eats small amounts of food and has not over-eaten or felt guilty or purges. After 3 months, patient has lost weight, is sleeping well and energetic. No adverse effects on 100 mg qhs.

### EXAMPLE 3

**Patient 03:** Patient very depressed, thoughts of suicide for years, currently on topiramate and has been purging less than 1/day which is a decrease. At this visit, increased topiramate to 100 mg qhs (not sure what dose was before this) as seems to have decreased purging. Two weeks later, patient is more depressed, sedated and purging more; changing depression medicines now. Two days later, patient presents urgently as throwing up uncontrollably due to stress and apprehension. Patient also has migraines which cause emesis. Patient admitted to hospital for 4 days. After hospitalization, mood is improved. Topiramate increased to 175 mg/day (100 mg qhs and 25 mg tid 1 hour prior to meals). Two weeks later, her mood is much improved and purging only once every other day which is a marked decrease.

### EXAMPLE 4

**Patient 04:** Patient depressed and purging 5/day - lots of family issues and lot of weight gain from current medicines - admitted to hospital for 2 weeks and taking topiramate 50 mg qhs. Topiramate was increased to 200 mg qhs while in hospital. Three weeks after hospitalization, patient is sleeping better, not purging and has lost 30 lbs. Two weeks later, patient continues doing well, sleeping well and happy about weight loss; no adverse effects. One month later, continues doing well, has not binged and has lost the 30 lbs about which she is very happy. She continues topiramate at 200 mg qhs.

## Claims

1. The use of a compound for the manufacture of a medicament for treating bulimia nervosa in a subject, said compound being of formula I: 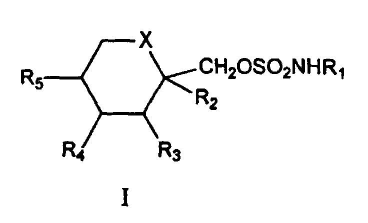 wherein
X is CH₂ or oxygen;
R₁ is hydrogen or C₁-C₄ alkyl; and
R₂, R₃, R₄ and R₅ are independently hydrogen or C₁-C₃ alkyl and, when X is CH₂, R₄ and
R₅ may be alkene groups joined to form a benzene ring and, when X is oxygen, R₂ and R₃ and/or R₄ and R₅ together may be a methylenedioxy group of the following formula (II): 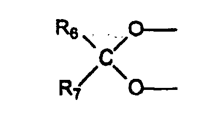
wherein R₆ and R₇ are the same or different and are hydrogen, C₁-C₃ alkyl or R₆ and R₇ together with the carbon to which they are attached are joined to form a cyclopentyl or cyclohexyl ring.

2. The use of a compound of formula I, as defined in claim 1, for the manufacture of a medicament for decreasing bingeing and purging episodes in a subject suffering from bulimia nervosa.

3. The use of claim 1 or claim 2 wherein the compound of formula I is topiramate.

4. The use of claim 1 or claim 2 wherein the compound of formula I is administrable in an amount from 15 mg to 500 mg per day.

5. The use of claim 1 or claim 2 wherein the compound of formula I is administrable in an amount from 16 mg to 200 mg per day.

## Patentansprüche

1. Verwendung einer Verbindung zur Herstellung eines Medikaments zur Behandlung von Bulimia nervosa bei einem Patienten, wobei die Verbindung die Formel I hat: 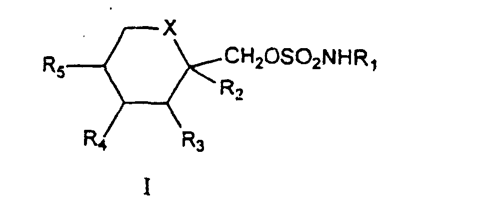 wobei
X CH₂ oder Sauerstoff ist;
R₁ Wasserstoff oder C₁-C₄-Alkyl ist; und
R₂, R₃, R₄ und R₅ unabhängig Wasserstoff oder C₁-C₃-Alkyl sind und, wenn X CH₂ ist, R₄ und R₅ Alkengruppen sein können, die miteinander verbunden sind, um einen Benzolring zu bilden, und, wenn X Sauerstoff ist, R₂ und R₃ und/oder R₄ und R₅ zusammen eine Methylendioxygruppe der folgenden Formel (II) sind: 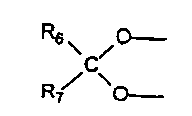
wobei R₆ und R₇ dieselben oder unterschiedlich sind und Wasserstoff, C₁-C₃-Alkyl sind, oder wobei R₆ und R₇ zusammen mit dem Kohlenstoff, an den sie angehängt sind, verbunden sind, um einen Cyclopentyl- oder Cyclohexyl-Ring zu bilden.

2. Verwendung einer Verbindung der Formel I, wie in Anspruch 1 definiert, zur Herstellung eines Medikaments zur Verringerung der Schling- und Entleerungsepisoden bei einem Patienten, der an Bulimia nervosa leidet.

3. Verwendung nach Anspruch 1 oder Anspruch 2, wobei die Verbindung der Formel I Topiramat ist.

4. Verwendung nach Anspruch 1 oder Anspruch 2, wobei die Verbindung der Formel I in einer Menge von 15mg bis 500 mg pro Tag verabreichbar ist.

5. Verwendung nach Anspruch 1 oder Anspruch 2, wobei die Verbindung der Formel I in einer Menge von 16 mg bis 200 mg pro Tag verabreichbar ist.

## Revendications

1. Utilisation d'un composé pour la fabrication d'un médicament pour le traitement de la boulimie nerveuse chez un sujet, ledit composé étant de la formule I : 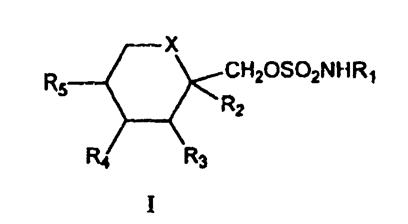 où
X est CH₂ ou oxygène ;
R₁ est hydrogène ou alkyle C₁-C₄ ; et
R₂, R₃, R₄ et R₅ sont indépendamment hydrogène ou alkyle C₁-C₃ et, quand X est CH₂, R₄ et R₅ peuvent être des groupes alcènes joints pour former un cycle benzène et quand X est oxygène, R₂ et R₃ et/ou R₄ et R₅ peuvent ensemble être un groupe méthylènedioxy de la formule (II) qui suit : 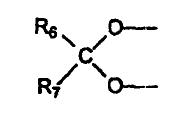
où R₆ et R₇ sont identiques ou différents et sont hydrogène, alkyle C₁-C₃ ou bien R₆ et R₇ avec le carbone auquel ils sont attachés sont joints pour former un cycle cyclopentyle ou cyclohexyle.

2. Utilisation d'un composé de la formule I, tel que défini à la revendication 1, pour la fabrication d'un médicament pour diminuer les épisodes de vomissements et de purge chez un sujet souffrant de boulimie nerveuse.

3. Utilisation de la revendication 1 ou de la revendication 2, où le composé de la formule I est topiramate.

4. Utilisation de la revendication 1 ou de la revendication 2, où le composé de formule I est administrable en une quantité de 15 mg à 500 mg par jour.

5. Utilisation de la revendication 1 ou de la revendication 2, où le composé de la formule I est administrable en une quantité de 16 mg à 200 mg par jour.
